# FASCICULE DE BREVET EUROPEEN

(11) **EP 4 619 380 B1**
(45) Date de publication et mention de la délivrance du brevet: **12.08.2026**
(21) Numéro de dépôt: 23806301.0
(22) Date de dépôt: 16.11.2023
(51) Int. Cl.: C07C 233/25, C07C 231/02

(54) **PROCÉDÉ DE PRÉPARATION D'UNE AMINE PRIMAIRE AROMATIQUE N-ACÉTYLÉE**
VERFAHREN ZUR HERSTELLUNG EINES N-ACETYLIERTEN AROMATISCHEN PRIMÄREN AMINS
METHOD FOR THE PREPARATION OF AN N-ACETYLATED AROMATIC PRIMARY AMINE

(30) Priorité: 17.11.2022 FR 2211970
(43) Date de publication de la demande: 24.09.2025
(73) Titulaire: Novacyl, 69134 Ecully Cedex 09 (FR)
(72) Inventeur: N GOMPAZA-DIARRA, Joannah, 69009 LYON (FR); COMBET, Jean-Paul, Antoine, 69005 LYON (FR); JACQUEMET, Léonard, Louis, Antoine, 69003 LYON (FR); SEIGNEURET, Rémy, Claude, Roger, 38150 ASSIEU (FR)
(74) Mandataire: Lavoix
(86) Numéro de dépôt international: PCT/EP2023/082102
(87) Numéro de publication internationale: WO 2024/105187

(56) Documents cités:
- EP-A1- 0 633 874
- EP-B1- 0 633 874
- US-A- 3 781 354
- US-A1- 2012 065 423

## Description

La présente invention concerne un procédé de préparation en continu d'une amine primaire aromatique N-acétylée, notamment d'un N-acétyl-aminophénol, de préférence de paracétamol, également appelé N-acétyl-p-aminophénol (APAP), N-(4-hydroxyphényl)acétamide ou acétaminophène.

Le N-acétyl-aminophénol comprend plusieurs régioisomères, dont le paracétamol. Les propriétés antalgiques et antipyrétiques du paracétamol en font une substance active de choix depuis plus d'un siècle.

La préparation de N-acétyl-aminophénol a été largement étudiée. De nombreux procédés de synthèse ont été décrits typiquement à partir du phénol, du chlorobenzène ou encore du nitrobenzène.

Plusieurs procédés ont prouvé leur efficacité à l'échelle industrielle. Parmi eux, Celanese a développé une voie dont la dernière étape est un réarrangement de Beckmann de l'oxime de la p-hydroxyacétophénone.

Dans les autres procédés industriels, la dernière étape est la N-monoacétylation du p-aminophénol (noté par la suite PAP). La monoacétylation du PAP peut avoir lieu en présence d'anhydride acétique ou d'acide acétique.

Utiliser l'acide acétique dans cette réaction implique une température de réaction élevée, un temps de séjour élevé qui conduit à la production d'impuretés indésirables dans le produit final.

De nombreux documents de l'art antérieur indiquent également que l'emploi d'anhydride acétique présenterait certains désagréments, notamment une pureté insuffisante et une coloration du produit final.Ils ont ainsi pour objet de remédier à ce problème. On peut notamment citer parmi eux les brevets US 3,042,719; US 3,748,358; US 3,781,354 ou encore US 4,264,526.

US 2012/065423 vise à la préparation en continu des amines primaires aromatiques N-acétylées.

La présence d'impuretés, en particulier colorées, entraine une purification longue et la production de nombreux déchets et effluents.

Par ailleurs, dans l'art antérieur, la réaction de monoacétylation du PAP a lieu en batch, ce qui implique une productivité limitée au niveau industriel.

Dans le contexte environnemental actuel, il est nécessaire de développer un procédé de préparation d'amine primaire aromatique N-acétylée, et de préférence de N-acétyl-aminophénol, avec une empreinte écologique fortement réduite tout en étant industriellement viable et avec une forte productivité.

A cette fin, l'invention a pour objet un procédé de préparation d'une amine primaire aromatique N-acétylée comprenant les étapes de :
b) mise en contact d'une amine primaire aromatique avec un agent d'acétylation choisi parmi l'anhydride acétique, un halogénure d'acétyle et un mélange de ceux-ci en quantité équimolaire ou submolaire par rapport à l'amine primaire aromatique, ce par quoi est obtenu un flux réactionnel comprenant une amine primaire aromatique N-acétylée, ladite mise en contact étant réalisée en l'absence de palladium,
c) refroidissement du flux réactionnel, ce par quoi l'amine primaire aromatique N-acétylée cristallise,
les étape b) et c) étant réalisées en continu.

Les inventeurs ont mis au point un procédé de préparation de N-acétyl-aminophénol avec un impact environnemental minimisé.

La mise en oeuvre des étapes b) et c) en continu permet notamment de réduire la quantité de déchets et d'effluents, tout en augmentant la productivité. Non seulement la réaction d'acétylation (étape b)), mais également la cristallisation (étape c)), ont lieu en continu.

L'amine primaire aromatique est de préférence un aminophénol, notamment le 2-aminophénol, le 3-aminophénol, ou le 4-aminophénol, de préférence le 2-aminophénol ou le 4-aminophénol. De manière particulièrement préférée, l'amine primaire aromatique est le 4-aminophénol (également nommé p-aminophénol (PAP)), et le procédé permet alors de préparer du paracétamol (APAP).

L'agent d'acétylation est choisi parmi l'anhydride acétique, un halogénure d'acétyle et un mélange de ceux-ci. En particulier, l'halogénure d'acétyle est le chlorure d'acétyle ou le bromure d'acétyle, plus particulièrement le chlorure d'acétyle. Typiquement, l'agent d'acétylation est l'anhydride acétique.

Le procédé peut comprendre, avant l'étape b), une étape a) de fourniture de l'amine primaire aromatique.

De préférence, l'amine primaire aromatique fournie à l'étape a) est à une température de 0°C à 120°C, notamment de 0°C à 110°C, en particulier de 5°C à 90°C. Cette température correspond à celle de l'amine primaire aromatique juste avant la mise en contact de l'étape b). Des températures supérieures sont évitées afin de ne pas dégrader l'amine primaire aromatique.

Le degré de pureté de l'amine primaire aromatique est alors typiquement d'au moins 95%, de préférence au moins 97%, plus préférentiellement au moins 98%.

Dans un premier mode de réalisation, l'amine primaire aromatique fournie à l'étape a) est sous forme essentiellement exempte de solvant. Généralement, l'amine primaire aromatique fournie est sous forme de solide, typiquement sous forme de poudre.

Dans ce mode de réalisation, la température de l'amine primaire aromatique à l'étape a) est généralement de 0°C à 85°C, de préférence de 5°C à 80°C, plus préférentiellement de 20°C à 70°C. Cette température correspond à celle de l'amine primaire aromatique juste avant la mise en contact de l'étape b).

Dans un deuxième mode de réalisation, l'amine primaire aromatique fournie à l'étape a) est sous forme de mélange de l'amine primaire aromatique et d'un solvant. Ce mélange sera utilisé à l'étape b) à titre de source d'amine primaire aromatique.

L'étape a) peut comprendre une étape a1) de mélange d'une amine primaire aromatique et d'un solvant pour obtenir un mélange d'une amine primaire aromatique et d'un solvant, puis éventuellement une étape a2) de mise de ce mélange à une température allant de 20°C à 120°C, notamment de 20°C à 110°C, en particulier de 40°C à 90°C. Cette température correspond à celle du mélange juste avant la mise en contact de l'étape b). De préférence, la température est inférieure au point d'ébullition du solvant, notamment au moins 5°C inférieure au point d'ébullition du solvant.

Le mélange d'une amine primaire aromatique et d'un solvant peut être sous forme de solution ou de suspension. La forme du mélange dépend notamment de la concentration massique en amine primaire aromatique dans le solvant et de la température du mélange. En particulier il s'agit d'une suspension. Alternativement, il s'agit d'une solution.

Le solvant peut être de l'eau, de l'acide acétique ou un mélange de ceux-ci. Les proportions en acide acétique/eau peuvent varier de 100/0 à 0/100, notamment de 90/10 à 10/90, par exemple de 75/25 à 25/75. En particulier, le solvant est de l'eau. Avantageusement, le solvant est de l'acide acétique.

La concentration massique en amine primaire aromatique dans le mélange peut être de 10% à 50%, soit de 0,10 kg à 0,50 kg d'amine primaire aromatique dans 1 kg du mélange amine primaire aromatique-solvant. De préférence, elle est de 15% à 45%, plus préférentiellement de 20% à 45%.

L'étape a) peut être réalisée en continu. En particulier, les étapes a1) et a2) sont réalisées en continu.

Le procédé peut comprendre, avant l'étape b), une étape a') de fourniture d'un agent d'acétylation choisi parmi l'anhydride acétique, un halogénure d'acétyle et un mélange de ceux-ci.

De préférence, l'agent d'acétylation fourni à l'étape a') est à une température de -10°C à son point d'ébullition, en particulier de 0°C à son point d'ébullition, plus particulièrement de 5°C à son point d'ébullition. Lorsque l'agent d'acétylation est un mélange, il est de préférence fourni à une température inférieure ou égale au point d'ébullition le plus faible des agents d'acétylation qu'il contient. Cette température correspond à celle de l'agent d'acétylation juste avant la mise en contact de l'étape b). Des températures supérieures sont évitées afin de ne pas dégrader l'agent d'acétylation. Selon une première alternative, l'agent d'acétylation est à la température extérieure, typiquement entre -10 à 40°C. En effet, le stock d'agent d'acétylation est généralement conservé à l'extérieur. Selon une deuxième alternative, l'agent d'acétylation est à la même température que celle de l'amine primaire aromatique.

Le degré de pureté de l'agent d'acétylation est typiquement d'au moins 95%, de préférence au moins 97%, plus préférentiellement au moins 98%.

Dans un premier mode de réalisation, l'agent d'acétylation fourni est sous forme essentiellement exempte de solvant.

Dans un deuxième mode de réalisation, l'agent d'acétylation fourni est sous forme de mélange d'agent d'acétylation et d'un solvant. Ce mélange d'agent d'acétylation et d'un solvant sera utilisé à l'étape b) à titre de source d'agent d'acétylation.

L'étape a') peut comprendre une étape a1') de mélange d'agent d'acétylation et d'un solvant pour obtenir un mélange d'agent d'acétylation et d'un solvant, puis éventuellement une étape a2') de mise de ce mélange à une température allant de 20°C à 110°C, en particulier de 40°C à 90°C. Cette température correspond à celle du mélange d'agent d'acétylation et d'un solvant juste avant la mise en contact de l'étape b). De préférence, la température est inférieure au point d'ébullition du solvant, notamment au moins 5°C inférieure au point d'ébullition du solvant.

Le mélange d'agent d'acétylation et de solvant est sous forme de solution à la température à laquelle il est utilisé pour réaliser l'étape b).

Le solvant est de préférence de l'acide acétique.

La concentration massique en agent d'acétylation dans le mélange peut être de 20% à 98%, soit de 0,20 kg à 1 kg d'agent d'acétylation dans 1 kg du mélange agent d'acétylation-solvant. De préférence, elle est de 30% à 95%, plus préférentiellement de 40% à 90%.

L'étape a') peut être réalisée en continu. En particulier, les étapes a1') et a2') sont réalisées en continu.

Le procédé comprend une étape b) de mise en contact de l'amine primaire aromatique avec de l'agent d'acétylation en quantité équimolaire ou submolaire par rapport à l'amine primaire aromatique, ce par quoi est obtenu un flux réactionnel comprenant une amine primaire aromatique N-acétylée, ladite mise en contact étant réalisée en l'absence de palladium.

Le procédé met en oeuvre une quantité équimolaire ou submolaire d'agent d'acétylation par rapport à l'amine primaire aromatique. Généralement, le nombre d'équivalents d'agent d'acétylation par rapport à l'amine primaire aromatique est inférieur ou égal à 1,00, de préférence de 0,80 à 1,00, par exemple de 0,90 à 1,00, notamment de 0,95 à 1,00. Le contrôle de la quantité d'agent d'acétylation en continu permet d'obtenir à l'issue du procédé un produit plus propre et moins coloré. Ceci permet de simplifier la purification de la N-amine primaire aromatique N-acétylée obtenue.

Notamment à l'étape b), la quantité d'agent d'acétylation est équimolaire par rapport à l'amine primaire aromatique.

L'étape b) est réalisée en l'absence de palladium, de préférence en l'absence de catalyseur d'hydrogénation, voire même en l'absence de catalyseur métallique sous forme de métal ou d'oxyde métallique. Ainsi, il n'y a pas de réaction d'hydrogénation en même temps que l'acétylation, ce qui différencie le procédé selon l'invention de ceux mettant en oeuvre une hydrogénation d'un composé nitro-aromatique (en particulier un nitrophénol, tel que le para-nitrophénol) pour obtenir une amine primaire aromatique qui est acétylée de façon concomitante. De préférence, l'étape b) est réalisée en l'absence d'hydrogène.

De préférence, l'étape b) est réalisée dans un solvant. Ce solvant peut être ajoutée lors de l'étape b). Le solvant est typiquement de l'eau, de l'acide acétique ou un mélange de ceux-ci, en particulier de l'acide acétique. De préférence, lorsque l'amine primaire aromatique fournie à l'étape a) est sous forme essentiellement exempte de solvant, le procédé comprend l'ajout d'un solvant lors de l'étape b). De préférence, lorsque l'amine primaire aromatique fournie à l'étape a) est sous forme de mélange de l'amine primaire aromatique et d'un solvant, aucun solvant n'est ajouté lors de l'étape b) (on entend par « aucun solvant », aucun solvant supplémentaire à celui du mélange amine primaire aromatique/solvant et du mélange agent d'acétylation/ solvant si l'agent d'acétylation est ajouté sous forme de mélange agent d'acétylation/ solvant).

La mise en contact de l'amine primaire aromatique et de l'agent d'acétylation lors de l'étape b) permet la réaction d'acétylation, et la formation d'un flux réactionnel comprenant une amine primaire aromatique N-acétylée, qui est le produit de l'acétylation de l'amine primaire aromatique. Généralement, l'acétylation est une monoacétylation et l'amine primaire aromatique N-acétylée est une amine primaire aromatique N-monoacétylée.

Généralement, l'étape b) est réalisée à une température de 25°C à 120°C, notamment de 25°C à 110°C, de préférence de 30°C à 100°C, plus préférentiellement de 35 °C à 95°C, par exemple de 40°C à 90°C. En deçà de 25°C, le flux réactionnel est généralement trop visqueux. Au-delà de 110°C, le taux d'impuretés produites est souvent trop élevé.

Généralement, l'étape b) dure moins de 2 heures, notamment moins de 1 heure, en particulier de 1 seconde à 45 minutes, plus préférentiellement de 1 minute à 30 minutes. Typiquement, plus la température est élevée, plus la durée de l'étape b) est réduite.

L'étape b) est généralement réalisée dans un réacteur. Le réacteur peut être un réacteur tubulaire ou un réacteur à cuve agitée en continu. Typiquement, le réacteur est un réacteur à cuve agitée en continu (couramment nommé CSTR).

L'amine primaire aromatique (éventuellement sous forme de mélange avec un solvant), l'agent d'acétylation (éventuellement sous forme de mélange avec un solvant) et l'éventuel solvant sont des flux d'entrée du réacteur. Au sein du réacteur, ces flux d'entrée sont mis en contact, ce qui permet la formation d'un flux réactionnel comprenant une amine primaire aromatique N-acétylée issue de la réaction d'acétylation. Ce flux réactionnel est ensuite sorti du réacteur.

La durée de l'étape b) décrite ci-dessus est typiquement le temps de séjour dans le réacteur.

Le flux réactionnel au sein du réacteur ou en sortie de réacteur peut être sous forme de solution ou de suspension. Lorsqu'il est sous forme de suspension, le procédé peut comprendre, après l'étape b), une ou deux étape(s) additionnelle(s) b1) de chauffage du flux réactionnel afin de former une solution, et optionnellement une étape b2) de filtration du flux réactionnel (étant entendu que c'est le filtrat qui sert pour la suite du procédé). Lorsque le flux réactionnel est sous forme de solution, le procédé peut comprendre, après l'étape b), une étape additionnelle b2) de filtration du flux réactionnel (étant entendu que c'est le filtrat qui sert pour la suite du procédé).

Lors de l'étape b1), le flux réactionnel peut être chauffé à une température allant de 60°C à 120°C, de préférence de 70°C à 110°C. En particulier, le flux réactionnel est chauffé au moins 10°C de plus que la température de l'étape b), plus particulièrement au moins 20°C de plus, notamment entre 10 et 50°C de plus, par exemple entre 10 et 30°C de plus.

Généralement, l'étape b1) dure moins de 2 heures, notamment moins de 1 heure, en particulier de 1 seconde à 45 minutes, plus préférentiellement de 1 minute à 30 minutes. Typiquement, plus la température est élevée, plus la durée de l'étape b1) est réduite.

L'étape b1) est généralement réalisée dans un réacteur. Le réacteur peut être un réacteur tubulaire ou un réacteur à cuve agitée en continu. Typiquement, le réacteur est un réacteur à cuve agitée en continu (couramment nommé CSTR).

Le procédé comprend une étape c) de refroidissement du flux réactionnel, ce par quoi l'amine primaire aromatique N-acétylée cristallise. Lors de cette étape c), le flux réactionnel est généralement refroidi à une température de 15°C à 35°C, en particulier de 20°C à 30°C. Typiquement, il s'agit de la température ambiante.

De préférence, lorsqu'à l'étape b), l'agent d'acétylation est en quantité submolaire par rapport à l'amine primaire aromatique, lors de l'étape c), de l'agent d'acétylation est ajouté au flux réactionnel. Cet ajout d'agent d'acétylation permet de minimiser la formation de produits secondaires indésirables et donc d'améliorer la pureté de l'amine primaire aromatique N-acétylée obtenue par le procédé. Il permet d'améliorer la conversion finale et de limiter la coloration.

La quantité d'agent d'acétylation ajoutée lors de l'étape c) est de préférence telle que la quantité totale d'agent d'acétylation ajoutée aux étapes b) et c) est au moins de 1,00 équivalent molaire, notamment de 1,00 à 1,15 équivalents molaires, de préférence de 1,00 à 1,08 équivalents molaires, de manière particulièrement préférée de 1,00 équivalent molaire, par rapport à l'amine primaire aromatique utilisée à l'étape b).

Lors de l'étape c), l'agent d'acétylation est de préférence ajouté au flux réactionnel lorsque la température dudit flux réactionnel est inférieure à 70°C, notamment inférieure à 60°C, de préférence inférieure à 55°C, plus préférentiellement inférieure ou égale à 40°C. Typiquement, l'agent d'acétylation est ajouté au flux réactionnel lorsque la température du flux réactionnel est supérieure à 15°C. Ces plages de températures permettent avantageusement de minimiser la formation de produits secondaires indésirables. L'étape c) comprend alors typiquement le refroidissement du flux réactionnel issu de l'étape b) à une température inférieure à 80°C, notamment inférieure à 60°C, de préférence inférieure à 55°C, plus préférentiellement inférieure ou égale à 40°C, puis l'ajout au flux réactionnel d'agent d'acétylation.

De préférence, l'agent d'acétylation ajouté lors de l'étape c) est à une température de 0°C à 90°C, plus particulièrement de 5 à 50°C. Des températures supérieures sont évitées afin de ne pas dégrader l'agent d'acétylation et/ou ne pas former de produits indésirables.

Selon une première alternative, l'agent d'acétylation est à la température extérieure, typiquement entre -10 à 40°C. En effet, le stock d'agent d'acétylation est généralement conservé à l'extérieur.

Selon une deuxième alternative, l'agent d'acétylation est à la même température que le flux réactionnel à la sortie de l'étape b).

Selon une troisième alternative, l'agent d'acétylation est à la même température que la température du flux réactionnel lorsque l'agent d'acétylation est ajouté. Par exemple, l'agent d'acétylation est à une température comprise entre 15 et 60°C lorsqu'il est ajouté au flux réactionnel, lui-même à une température comprise entre 15 et 60°C.

Le degré de pureté de l'agent d'acétylation est typiquement d'au moins 95%, de préférence au moins 97%, plus préférentiellement au moins 98%.

Dans un premier mode de réalisation, l'agent d'acétylation ajouté lors de l'étape c) est sous forme essentiellement exempte de solvant. Généralement, l'agent d'acétylation est sous forme liquide.

Dans un deuxième mode de réalisation, l'agent d'acétylation ajouté lors de l'étape c) est sous forme de mélange de l'agent d'acétylation et d'un solvant. C'est ce mélange qui est alors ajouté lors de l'étape c) à titre de source d'agent d'acétylation.

L'étape c) peut comprendre une étape c1) de mélange d'agent d'acétylation et d'un solvant pour obtenir un mélange d'agent d'acétylation et d'un solvant, puis éventuellement une étape c2) de mise de ce mélange à la température à laquelle il est ajouté lors de l'étape c). Le mélange d'agent d'acétylation et de solvant peut être sous forme de solution à la température à laquelle il est ajouté lors de l'étape c).

Le solvant est de préférence de l'acide acétique.

La concentration massique en agent d'acétylation dans le mélange peut être de 20% à 98%, soit de 0,20 kg à 1 kg d'agent d'acétylation dans 1 kg du mélange agent d'acétylation-solvant. De préférence, elle est de 30% à 95%, plus préférentiellement de 40% à 90%.

Dans un premier mode de réalisation, l'étape c) comprend le transfert du flux réactionnel dans une succession de réacteurs à cuve agitée en continu (CSTR), en particulier au moins deux CSTR, plus particulièrement au moins trois CSTR. De préférence, la succession de CSTR va de deux CSTR à dix CSTR, plus préférentiellement de trois CSTR à huit CSTR. Par une succession de CSTR, on entend des CSTR installés en série.

Le temps de séjour du flux réactionnel dans chaque CSTR est typiquement de 15 minutes à 1 heure.

Dans un deuxième mode de réalisation, l'étape c) comprend le transfert du flux réactionnel dans au moins un échangeur tubulaire, de préférence d'un à trente échangeur(s) tubulaire(s) en série. L'échangeur tubulaire, ou chaque échangeur tubulaire indépendamment des autres lorsqu'il y en a plusieurs, peut être un échangeur tubulaire avec ou sans système de transport.

Le temps de séjour dans l'échangeur tubulaire ou la série d'échangeurs tubulaires est typiquement de 15 minutes à 1 heure.

Dans un troisième mode de réalisation, l'étape c) comprend le transfert du flux réactionnel dans au moins un échangeur tubulaire et au moins un CSTR, notamment au moins un échangeur tubulaire puis au moins un CSTR en série. Plus particulièrement l'étape c) comprend le transfert du flux réactionnel dans une série d'un à trente échangeurs tubulaires puis dans une série d'un à dix CSTR. L'échangeur tubulaire, ou chaque échangeur tubulaire indépendamment des autres lorsqu'il y en a plusieurs, peut être un échangeur tubulaire avec ou sans système de transport. Le temps de séjour dans l'échangeur tubulaire ou dans la série d'échangeurs tubulaires est typiquement de 15 minutes à 1 heure et/ou le temps de séjour dans chaque CSTR est typiquement de 15 minutes à 1 heure.

Le procédé comprend généralement, après l'étape c), une étape de récupération de l'amine primaire aromatique N-acétylée. Cette étape de récupération de l'amine primaire aromatique N-acétylée peut comprendre une étape c3) de filtration du flux réactionnel et optionnellement une étape c4) de lavage de l'amine primaire aromatique N-acétylée avec le même solvant que les étapes b) et c) ou avec un autre solvant. Typiquement, l'autre solvant est choisi parmi l'acide acétique, l'eau ou un mélange de ceux-ci.

Le procédé peut comprendre ou être exempt d'étape supplémentaire de purification de l'amine primaire aromatique N-acétylée après l'étape c). Par exemple, le procédé peut comprendre, après l'étape c), une étape d) de recristallisation de l'amine primaire aromatique N-acétylée, en particulier une recristallisation dans l'eau, dans l'acide acétique ou dans un mélange de ceux-ci, en particulier dans l'eau.

Généralement, le procédé ne comprend qu'une unique recristallisation de l'amine primaire aromatique N-acétylée. Alternativement, l'étape d) de recristallisation peut être répétée.

Typiquement, l'étape d) est réalisée en continu.

Le procédé comprend généralement, après l'étape d), une étape de récupération de l'amine primaire aromatique N-acétylée recristallisée. Cette étape de récupération de l'amine primaire aromatique N-acétylée recristallisée peut comprendre une étape d1) de filtration et optionnellement une étape d2) de lavage de l'amine primaire aromatique N-acétylée avec le même solvant que l'étape d) ou avec un autre solvant. Typiquement, l'autre solvant est choisi parmi l'acide acétique, l'eau ou un mélange de ceux-ci.

Le procédé peut comprendre, après l'étape c), notamment après l'étape c3) ou c4) ou après l'étape d), notamment après l'étape d1) ou d2) si elle(s) est(sont) présente(s), une étape e) d'évaporation des solvants restants dans l'amine primaire aromatique N-acétylée.

L'amine primaire aromatique N-acétylée obtenue à l'issue du procédé se présente sous forme de cristaux. Dans le cas de l'APAP, la structure cristalline obtenue est le polymorphe de type I.

Typiquement, dans le cas de l'APAP, à l'issue de l'étape e), la taille des cristaux est telle que le D90 est supérieur à 200 µm, en particulier supérieur à 250 µm, plus particulièrement compris entre 250 et 700 µm (déterminé par granulométrie laser).

Le procédé peut comprendre, après l'étape e), une étape f1) de broyage de l'amine primaire aromatique N-acétylée et/ou une étape f2) de tamisage de l'amine primaire aromatique N-acétylée.

L'amine primaire aromatique N-acétylée obtenue à la suite de l'étape c), notamment après l'étape c3) ou c4), ou suite à l'étape d), notamment après l'étape d1) ou d2), est telle que la quantité d'impuretés est inférieure à 0,5% en poids, en particulier inférieure à 0,2% en poids. Lorsque l'amine primaire aromatique est un aminophénol, les impuretés principales sont l'aminophénol (produit de départ n'ayant pas réagi) et l'aminophénol N-acétylé et O-acétylé. La quantité en aminophénol (produit de départ) est de préférence inférieure à 100 ppm, plus préférentiellement inférieure à 50 ppm. Avantageusement, le pourcentage en aminophénol N-acétylé et O-acétylé est inférieur à 0,1%, par exemple inférieur à 0,05%.

Lorsque l'amine primaire aromatique N-acétylée est l'APAP, sa couleur est blanche à blanc-cassé.

L'invention est illustrée par les exemples qui suivent.

### EXEMPLE 1 (invention) :

Le 4-aminophénol, l'anhydride acétique et l'acide acétique sont ajoutés de manière concomitante par des flux séparés dans un premier réacteur CSTR.

Le 4-aminophénol (PAP) est ajouté à température ambiante à un débit de 3980 g/h.

L'acide acétique est ajouté à température ambiante (20°C) à un débit de 7039 g/h.

0,96 eq. d'anhydride acétique (AA) est ajouté avec un débit de 3556 g/h (étape b)), ce par quoi l'acétylation se produit pour former du paracétamol (APAP).

Le premier réacteur CSTR est à une température de 80°C avec un temps de séjour du flux réactionnel de 1 minute.

Le flux réactionnel est ensuite transféré dans un second réacteur CSTR qui est à une température de 100°C avec un temps de séjour du flux réactionnel de 10 minutes (étape b1).

Le flux réactionnel est filtré (étape b2).

Il est ensuite transféré dans un échangeur tubulaire de type COBR (échangeur tubulaire à contre-pâle oscillatoire) avec un temps de résidence de 20 minutes et une température de sortie de 25°C (étape c).

Le flux réactionnel est transféré dans un troisième réacteur CSTR.

0,06 éq. d'AA est ajouté dans le troisième réacteur CSTR.

Le troisième réacteur CSTR est à une température de 25°C avec un temps de séjour du flux réactionnel additionnel de 4 heures.

L'APAP est ensuite filtré (étape c3), lavé avec au moins 0,3 vol. d'acide acétique et optionnellement au moins 0,5 vol. d'eau (étape c4).

Le rendement obtenu est de 70%. Le taux d'impuretés total est inférieur à 0,2% en poids.

### EXEMPLE 2 (invention) :

Le 4-aminophénol, l'anhydride acétique et l'acide acétique sont ajoutés de manière concomitante par des flux séparés dans un premier réacteur CSTR.

Le 4-aminophénol (PAP) est ajouté à température ambiante (20°C) à un débit de 4218 g/h.

L'acide acétique est ajouté à température ambiante à un débit de 6390 g/h.

0,98 eq d'anhydride acétique (AA) est ajouté avec un débit de 3822 g/h (étape b)), ce par quoi l'acétylation se produit pour former du paracétamol (APAP).

Le premier réacteur CSTR est à une température de 80°C avec un temps de séjour du flux réactionnel de 18 minutes.

Le flux réactionnel est ensuite transféré dans un second réacteur CSTR qui est à une température de 105°C avec un temps de séjour du flux réactionnel de 10 minutes (étape b1).

Le flux réactionnel est filtré (étape b2).

Il est ensuite transféré dans un échangeur tubulaire à surface raclée avec un temps de résidence de 20 minutes et une température de sortie de 45°C (étape c).

L'APAP est ensuite filtré (étape c3), lavé avec au moins 0,3 vol. d'acide acétique (étape c4).

Le rendement obtenu est de 65%. Le taux d'impuretés totales est inférieur à 0,2% en poids.

L'APAP est ensuite recristallisé dans l'eau (étape d).

Le rendement de recristallisation est de 93%. Le taux d'impuretés totales est inférieur à 0,2% en poids.

### EXEMPLE 3 (comparatif avec l'agent d'acétylation en excès par rapport à l'amine primaire aromatique) :

Le 4-aminophénol qui est dissous dans l'acide acétique à 70°C et l'anhydride acétique sont ajoutés de manière concomitante par des flux séparés dans un CSTR.

1,05 eq d'anhydride acétique (AA) est ajouté (étape b)), ce par quoi l'acétylation se produit pour former du paracétamol (APAP).

Le réacteur CSTR est à une température de 80°C avec un temps de séjour du flux réactionnel de 20 minutes.

Le flux réactionnel est ensuite transféré dans un autre réacteur CSTR qui est à une température de 105°C avec un temps de séjour du flux réactionnel de 20 minutes (étape b1).

Le flux réactionnel est filtré (étape b2).

Il est ensuite transféré dans un échangeur tubulaire à surface raclée avec un temps de résidence de 20 minutes et une température de sortie de 70°C (étape c).

Le flux réactionnel est transféré dans un réacteur CSTR et refroidi jusque 20°C. L'APAP est ensuite filtré (étape c3), lavé avec au moins 0,3 vol. d'acide acétique (étape c4).

Le rendement obtenu est de 72%. Le taux d'impuretés totales est de 0,4% en poids, donc deux fois plus élevé que ceux des exemples 1 et 2 selon l'invention.

## Revendications

1. Procédé de préparation d'une amine primaire aromatique N-acétylée comprenant les étapes de :
b) mise en contact d'une amine primaire aromatique avec un agent d'acétylation choisi parmi l'anhydride acétique, un halogénure d'acétyle et un mélange de ceux-ci en quantité équimolaire ou submolaire par rapport à l'amine primaire aromatique, ce par quoi est obtenu un flux réactionnel comprenant une amine primaire aromatique N-acétylée, ladite mise en contact étant réalisée en l'absence de palladium,
c) refroidissement du flux réactionnel, ce par quoi l'amine primaire aromatique N-acétylée cristallise,
les étapes b) et c)étant réalisées en continu.

2. Procédé selon la revendication 1, dans lequel l'amine primaire aromatique est un aminophénol, de préférence le 2-aminophénol, le 3-aminophénol, ou le 4-aminophénol.

3. Procédé selon la revendication 2, dans lequel l'amine primaire aromatique est le 4-aminophénol.

4. Procédé selon l'une quelconque des revendications 1 à 3, dans lequel, à l'étape b), l'agent d'acétylation est en quantité submolaire par rapport à l'amine primaire aromatique, et, lors de l'étape c), de l'agent d'acétylation est ajouté au flux réactionnel.

5. Procédé selon la revendication 4, dans lequel la quantité d'agent d'acétylation ajouté lors de l'étape c) est telle que la quantité totale d'agent d'acétylation ajouté aux étapes b) et c) est au moins de 1,00 équivalent molaire, notamment de 1,00 à 1,15 équivalents molaires, de préférence de 1,00 à 1,08 équivalents molaires, de manière particulièrement préférée de 1,00 équivalent molaire, par rapport à l'amine primaire aromatique utilisée à l'étape b).

6. Procédé selon la revendication 4 ou 5, dans lequel, lors de l'étape c), l'agent d'acétylation est ajouté au flux réactionnel lorsque la température dudit flux réactionnel est inférieure à 70°C, de préférence inférieure à 55°C.

7. Procédé selon l'une quelconque des revendications 1 à 6, comprenant, après l'étape c), une étape d) de recristallisation de l'amine primaire aromatique N-acétylée dans l'eau, dans l'acide acétique ou dans un mélange de ceux-ci.

8. Procédé selon l'une quelconque des revendications 1 à 7, dans lequel l'étape b) :
- est réalisée à une température de 25°C à 120°C, notamment de 25°C à 110°C, de préférence de 30°C à 100°C, plus préférentiellement de 40 °C à 90°C, et/ou
- dure moins de 2 heures, notamment moins de 1 heure, en particulier de 10 secondes à 45 minutes, plus préférentiellement de 1 minute à 30 minutes.

9. Procédé selon l'une quelconque des revendications 1 à 8, dans lequel l'étape b) est réalisée dans un solvant, de préférence dans l'acide acétique, l'eau ou un mélange de ceux-ci.

10. Procédé selon l'une quelconque des revendications 1 à 9, dans lequel l'étape b) est réalisée dans un réacteur à cuve agitée en continu.

## Patentansprüche

1. Verfahren zur Herstellung eines N-acetylierten primären aromatischen Amins, umfassend die Schritte:
b) Inkontaktbringen eines primären aromatischen Amins mit einem Acetylierungsmittel, ausgewählt aus Essigsäureanhydrid, einem Acetylhalogenid und einem Gemisch davon, in äquimolarer oder submolarer Menge bezogen auf das primäre aromatische Amin, wodurch ein Reaktionsstrom erhalten wird, der ein N-acetyliertes aromatisches primäres Amin umfasst, wobei das genannte Inkontaktbringen in Abwesenheit von Palladium durchgeführt wird,
c) Abkühlen des Reaktionsstroms, wodurch das N-acetylierte primäre aromatische Amin kristallisiert,
wobei die Schritte b) und c) kontinuierlich durchgeführt werden.

2. Verfahren nach Anspruch 1, wobei das primäre aromatische Amin ein Aminophenol ist, vorzugsweise 2-Aminophenol, 3-Aminophenol oder 4-Aminophenol.

3. Verfahren nach Anspruch 2, wobei das primäre aromatische Amin 4-Aminophenol ist.

4. Verfahren nach einem der Ansprüche 1 bis 3, wobei in Schritt b) das Acetylierungsmittel in submolarer Menge bezogen auf das primäre aromatische Amin vorliegt, und während Schritt c) Acetylierungsmittel zu dem Reaktionsstrom hinzugefügt wird.

5. Verfahren nach Anspruch 4, wobei die Menge an Acetylierungsmittel, die während Schritt c) hinzugefügt wird, derart ist, dass die Gesamtmenge an Acetylierungsmittel, die in den Schritten b) und c) hinzugefügt wird, mindestens 1,00 Moläquivalent, insbesondere 1,00 bis 1,15 Moläquivalente, vorzugsweise 1,00 bis 1,08 Moläquivalente, besonders bevorzugt 1,00 Moläquivalent, bezogen auf das in Schritt b) verwendete primäre aromatische Amin, beträgt.

6. Verfahren nach Anspruch 4 oder 5, wobei während Schritt c) das Acetylierungsmittel zu dem Reaktionsstrom hinzugefügt wird, wenn die Temperatur des genannten Reaktionsstroms niedriger als 70°C, vorzugsweise niedriger als 55°C, ist.

7. Verfahren nach einem der Ansprüche 1 bis 6, umfassend, nach Schritt c), einen Schritt d) der Umkristallisation des N-acetylierten primären aromatischen Amins in Wasser, in Essigsäure oder in einem Gemisch davon.

8. Verfahren nach einem der Ansprüche 1 bis 7, wobei Schritt b):
- bei einer Temperatur von 25°C bis 120°C, insbesondere von 25°C bis 110°C, vorzugsweise von 30°C bis 100°C, weiter bevorzugt von 40 °C bis 90°C, durchgeführt wird, und/oder
- weniger als 2 Stunden, insbesondere weniger als 1 Stunde, insbesondere von 10 Sekunden bis 45 Minuten, weiter bevorzugt von 1 Minute bis 30 Minuten, dauert.

9. Verfahren nach einem der Ansprüche 1 bis 8, wobei Schritt b) in einem Lösungsmittel durchgeführt wird, vorzugsweise in Essigsäure, Wasser oder einem Gemisch davon.

10. Verfahren nach einem der Ansprüche 1 bis 9, wobei Schritt b) in einem kontinuierlich betriebenen Rührkesselreaktor durchgeführt wird.

## Claims

1. A method for the preparation of an N-acetylated aromatic primary amine comprising the steps of:
b) bringing in contact an aromatic primary amine with an acetylating agent selected from acetic anhydride, an acetyl halide and a mixture thereof in an equimolar or sub-molar amount relative to the aromatic primary amine, whereby a reaction stream comprising an N-acetylated aromatic primary amine is obtained, the bringing in contact taking place in the absence of palladium,
c) cooling the reaction stream, whereby the N-acetylated aromatic primary amine crystallizes,
steps b) and c) being carried out continuously.

2. The method according to claim 1, wherein the aromatic primary amine is an aminophenol, preferably 2-aminophenol, 3-aminophenol, or 4-aminophenol.

3. The method according to claim 2, wherein the aromatic primary amine is 4-aminophenol.

4. The method according to any of claims 1 to 3, wherein in step b) the acetylating agent is in a sub-molar amount relative to the aromatic primary amine, and, during step c), acetylating agent is added to the reaction stream.

5. The method according to claim 4, wherein the amount of acetylating agent added in step c) is such that the total amount of acetylating agent added in steps b) and c) is at least 1.00 molar equivalents, in particular 1.00 to 1.15 molar equivalents, preferably 1.00 to 1.08 molar equivalents, particularly preferably 1.00 molar equivalents, with respect to the aromatic primary amine used in step b).

6. The method according to claim 4 or 5, wherein during step c) the acetylating agent is added to the reaction stream when the temperature of said reaction stream is less than 70°C, preferably less than 55°C.

7. The method according to any of claims 1 to 6 comprising, after step c), a step d) of recrystallization of a N-acetylated aromatic primary amine in water, in acetic acid or in a mixture thereof.

8. The method according to any of claims 1 to 7, wherein step b):
- is carried out at a temperature from 25°C to 120°C, in particular from 25°C to 110°C, preferably from 30°C to 100°C, more preferentially from 40°C to 90°C, and/or
- lasts less than 2 hours, in particular less than 1 hour, more particularly from 10 seconds to 45 minutes, more preferentially from 1 minute to 30 minutes.

9. The method according to any of claims 1 to 8, wherein step b) is carried out in a solvent, preferably acetic acid, water or a mixture thereof.

10. The method according to any of claims 1 to 9, wherein step b) takes place in a continuous stirred-tank reactor.
